**Europäisches Patentamt**

**European Patent Office**

**Office européen des brevets**

(19)

(11) Veröffentlichungsnummer: **0 259 468 B1**

(12) **EUROPÄISCHE PATENTSCHRIFT**

(45) Veröffentlichungstag der Patentschrift:
15.05.91 Patentblatt 91/20

(21) Anmeldenummer: 87902041.0

(22) Anmeldetag: 09.03.87

(86) Internationale Anmeldenummer:
PCT/DE87/00106

(87) Internationale Veröffentlichungsnummer:
WO 87/05294 11.09.87 Gazette 87/20

(51) Int. Cl.⁵: **C07C 405/00, C08B 37/16, A61K 31/557**

(54) **CYCLODEXTRINCLATHRATE VON CARBACYCLINDERIVATEN UND IHRE VERWENDUNG ALS ARZNEIMITTEL.**

Die Akte enthält technische Angaben, die nach dem Eingang der Anmeldung eingereicht wurden und die nicht in dieser Patentschrift enthalten sind.

(30) Priorität: 07.03.86 DE 3608088

(43) Veröffentlichungstag der Anmeldung:
16.03.88 Patentblatt 88/11

(45) Bekanntmachung des Hinweises auf die Patenterteilung:
15.05.91 Patentblatt 91/20

(84) Benannte Vertragsstaaten:
AT BE CH DE FR GB IT LI LU NL SE

(56) Entgegenhaltungen:
EP-A- 0 011 591
EP-A- 0 119 949
DE-A- 2 128 674
Chemical Abstracts, Band 96, Nr, 13, 29 März 1982 (Columbus, Ohio, USA), siehe Seite 680, Zusammenfassung Nr. 103964d

(73) Patentinhaber: SCHERING AKTIENGESELLSCHAFT Berlin und Bergkamen
Müllerstrasse 170/178 Postfach 65 03 11
W-1000 Berlin 65 (DE)

(72) Erfinder: SKUBALLA, Werner
Olwenstr. 13
W-1000 Berlin 28 (DE)
Erfinder: VORBRÜGGEN, Helmut
Wilkestr. 7
W-1000 Berlin 27 (DE)
Erfinder: DAHL, Helmut
Gollanczstr. 102
W-1000 Berlin 28 (DE)
Erfinder: STÜRZEBECHER, Claus-Steffen
Brigittenstr. 6a
W-1000 Berlin 46 (DE)
Erfinder: THIERAUCH, Karl-Heinz
Hochwildpfad 45
W-1000 Berlin 37 (DE)

## Beschreibung

Gegenstand der vorliegenden Erfindung sind Cyclodextrinclathrate von Carbacyclinanaloga und diese enthaltende Mittel. In der japanischen Offenlegungsschrift J 56 15 00 39 (s.Chem.Abstr. 1982, 96, 103964 d) werden allgemein Carbacyclin-Cyclodextrin-Einschlußverbindungen beschrieben. Carbacyclinanaloga sind pharmakologisch und medizinisch wertvolle Wirkstoffe, deren Herstellung und Anwendung beispielsweise in DE-OS 2845770, 3306123, 3226550 beschrieben sind. Diese Substanzen besitzen gegenüber dem entsprechenden natürlichen Prostacyclin bei ähnlichem Wirkungsspektrum eine wesentlich verbesserte Spezifität und vor allem wesentlich längere Wirkung.

Die in den erwähnten Offenlegungsschriften beschriebenen Carbacyclinanaloga liegen oft nicht kristallin vor, wodurch ihrer pharmazeutischen Anwendung Grenzen gesetzt sind. Hinzu kommt noch eine begrenzte Wasserlöslichkeit und Lösungsgeschwindigkeit.

Es wurde nun gefunden, daß Einschlußverbindungen dieser Carbacyclinanaloga mit Cyclodextrinen die genannten Nachteile nicht besitzen, d.h. ihre Wasserlöslichkeit wird verbessert, die Lösungsgeschwindigkeit wird gesteigert und die Einschlußverbindungen liegen in kristalliner Form vor. Außerdem wird ihre Stabilität beispielweise gegenüber Wärme, Licht und Sauerstoff erhöht und ihre galenische Zubereitung (Herstellen von Lösungen oder Tabletten) erleichtert.

Die Erfindung betrifft β-Cyclodextrin-Clathrate folgender Carbacyclinanaloga :

1) (5E)-(16S)-13,14-Didehydro-16,20-dimethyl-3-oxa-18,18,19,19-tetra-dehydro-6a-carba-prostaglandin-$I_2$

2) (5E)-(16S)-13,14-Didehydro-1a, 1b-dihomo-16,20-dimethyl-3-oxa-18,18 19,19-tetradehydro-6a-carba-prostaglandin-$I_2$

3) (5 E)-(16 S)-13,14,18,18,19,19-Hexadehydro-16,20-dimetbyl-6a-carbaprostaglandin-$I_2$

4) (5 E)-(16 RS)-16-Methyl-18,18,19,19-tetrade-hydro-6a-carba-prostaglandin-$I_2$

5) (5E)-(16S)-13,14-Didehydro-1a,1b-dihomo-16,20-dimethyl-18,18,19,19-tetradehydro-6a-carba-prostaglandin $I_2$

Besonders bevorzugte Verbindungen dieser Erfindung sind β-Cyclodextrinclathrate der oben genannten PG 3,-Derivate 1-3 bis.5 sowie lloprost-β-Cyclodextrinclathrat, das in JA 56 15 00 39 nicht namentlich genannt wird.

Zur Herstellung der erfindungsgemäßen Clathrate werden die Carbacyclinanaloga in einem pharmakologisch unbedenklichen Lösungsmittel, z.B. einem Alkohol, vorzugsweise Ethanol, einem Keton, z.B. Aceton oder einem Ether, z.B. Diethylether, aufgelöst und mit wäßrigen Lösungen von β-Cyclodextrin, bei 20-80°C vermischt, oder es werden die Säuren in Form der wäßrigen Lösungen ihrer Salze, z.B. der Natrium- oder Kaliumsalze mit dem Cyclodextrin versetzt und nach Lösung mit der äquivalenten Menge einer Säure, z.B. Salzäure oder Schwefelsäure, versetzt.

Hierbei oder nach dem Abkühlen kristallisieren die entsprechenden Clathrate aus. Man kann aber auch die öligen bzw. kristallinen Carbacycline durch längeres Rühren bei Raumtemperatur mit einer wäßrigen Lösung von Cyclodextrinen in entsprechende Kristallisate überführen. Die Clathrate können durch Absaugen und Trocknen als feste, frei fließende Kristalle isoliert werden.

Durch Wahl der geeigneten Cyclodextrin- und Wassermengen lassen sich die Clathrate in stöchiometrischer Zusammensetzung mit einem reproduzierbaren Wirkstoffgehalt erhalten.

Die entsprechend dieser Erfindung hergestellten Clathrate sind wertvolle Pharmaka.

Die Clathrate können in ihrer wasserfreien, hygroskopischen Form oder in einer wasserhaltigen, wenig hygroskopischen Form Verwendung finden.

Die neuen Cathacyclinclathenate liegen im stöchiometrischen Verhältnis von Carbacyclin : β-Cyclodextrin = 1 : 2 (3) vor.

Die Dosis der Verbindung ist 1-1.500 µg/kg/Tag, wenn sie am menschlichen Patienten verabreicht werden. Die Einheitsdosis für den pharmazeutisch akzeptablen Träger beträgt 0,01-100 mg.

Die Clathrate dieser Erfindung wirken blutdrucksenkend und bronchodilatorisch. Sie sind weiterhin geeignet zur Hemmung der Thrombozyten-Aggregation. Sie wirken cytoprotektiv am Magen, Darm, Herz, an der Leber, Niere und am Pankreas. Folglich stellen die neuen Cyclodextrin-Clathrate wertvolle pharmazeutische Wirkstoffe dar. Darüber hinaus weisen sie bei ähnlichem Wirkungsspektrum, verglichen mit entsprechenden Prostaglandinen, eine höhere Spezifität und vor allem eine wesentlich längere Wirksamkeit auf. Im Vergleich zu $PGI_2$ zeichnen sie sich durch größere Stabilität auf. Die hohe Gewebsspezifität der neuen Prostaglandine zeigt sich bei der Untersuchung an glattmuskulären Organen, wie zum Beispiel am Meerschweinchenileum oder an der isolierten Kaninchentrachea, wo eine wesentlich geringere Stimulation zu beobachten ist als bei der Applikation natürlicher Prostaglandine vom E-, A- oder F-Typ.

Die neuen Carbacyclin-Clathrate besitzen die für Prosta-cycline typischen Eigenschaften, wie zum Beispiel Senkung des peripheren arteriellen und koronaren vaskulären Widerstandes, Inhibierung der Thrombozytenaggregation und Auflösung von Plättchenthromben, myocardiale Zytoprotektion und damit Senkung des systemischen Blutdrucks ohne zugleich Schlagvolumen und koronare Durchblutung zu senken ; Behandlung von Schlaganfall, Prophylaxe und Therapie koronarer Herzerkrankungen, koronarer Thrombose, des Herzinfarkts, peripherer Arterienerkrankungen, Arteriosklerose und Thrombose, Prophylaxe und Therapie ischaemischer Attacken des ZNS-Systems, Therapie des Schocks, Inhibierung der Bronchlokonstriktion, Inhibierung der Magensäuresekretion, Zytoprotektion der Magen- und Darmschleimhaut, Zytoprotektionen in der Leber, Niere, Herz und im Pankreas, auch bei Organtransplantationen, antiallergische Eigenschaften, Senkung des pulmonalen vaskulären Widerstandes und des pulmonalen Blutdrucks, Förderung der Nierendurchblutung, Anwendung anstelle von Heparin oder als Adjuvans bei der Dialyse der Hämofiltration, Konservierung von Blutplasmakonserven, besonders von Blutplättchenkonserven, Inhibierung von Geburtswehen, Behandlung von Schwangerschaftstoxikose, Erhöhung der zerebralen Durchblutung etc. Außerdem besitzen die neuen Carbacyclinclathrate antiproliferative und antidiarrhoegene Eigenschaften.

Die Carbacyclinclathrate dieser Erfindung können auch in Kombination, zum Beispiel mit β-Blockern, Diuretika, Phosphondiesterasehemmern, Calciumantagonisten, nichtsteroidalen Entzündungshemmern, Leukotriensynthesehemmern, Leukotrienantagonisten, Thromboxansynthesehemmern oder Thromboxanantagonisten verwendet werden.

Die erfindungsgemäßen Clathrate können in flüssigen oder festen galenischen Formulierungen verwendet werden, wobei die Formulierungen enteral, parenteral, vaginal oder rektal verabreicht werden können, oder sie können auch in chirurgisches Nahtmaterial und in Kunststoffe eingebaut werden.

Zur Herstellung von Tabletten wird dar Prostaglandin-Cyclodextrin-Clathrat mit Trägersubstanzen und Hilfsstoffen, wie Laktose, Maisstärke, Polyvinylpyrrolidon und Magnesiumstearat, vermischt.

Zur Herstellung von Lösungen für die enterale und parenterale Anwendung werden die wäßrigen Cyclodextrin-ClathratLösungen zusammen mit Laktose lyophilisiert. Anschließend können die Lyophilisate mit physiologischer Kochsalzlösung auf die gewünschte Konzentration gebracht werden.

Die Erfindung umfaßt daher pharmazeutische Präparate und Formulierungen, die als Wirkstoff ein Cyclodextrin-Clathrat eines Carbacyclinanalogen enthalten.

Die Erfindung wird durch folgende Beispiele erläutert :

Beispiel 1

Man löst 560 mg β-Cyclodextrin in 4 ml Wasser bei 80°C, kühlt auf 60°C ab und tropft diese Lösung zu einer 60°C heißen Lösung von 18 mg (5E)-(16RS)-6-Methyl-18,18,19, 19-tetradehydro-6a-carbaprostaglandin-I₂ in 0,3 ml Äthanol. Man rührt 4 Stunden bei 60°C, 1 Stunde bei 45°C und 16 Stunden bei 25°C. Der ausgefallene Feststoff wird abgesaugt, mit 20 ml einer Mischung aus Wasser/Äthanol (1 : 1) gewaschen und 8 Stunden bei 0,1 Torr und 25°C über Phosphorpentoxid getrocknet. Man erhält 340 mg frei fließende Kristalle des β-Cyclodextrin-Clathrats des o.a. Carbacy clinanalogons.

Der Gehalt an Carbacyclinanalogon im Clathrat hurde durch Hochdruckflüssigkeitschromatographie bestimmt und betrug 4,23%.

Beispiel 2

Man rührt 1 g (5E)-(16S)-13,14-Didehydro-16,20-dimethyl-3-oxa-18,18,19,19-tetradehydro-6a-carba-prostaglandin-I₂ mit 30,3 g β-Cyclodextrin in 214 ml Wasser 48 Stunden bei 25°C. Der Feetstoff wird abgesaugt, mit 15 ml einer Mischung aus Wasser/Äthanol (1 : 1) gewaschen und 24 Stunden bei 0,1 Torr und 25°C über Phosphorpentoxid getrocknet. Man erhält 22,45 g frei fließende Kristalle des β-Cyclodextrin-Clathrats des o.a. Carbacyclinanalogons.

Der Gehalt an Carbacyclinanalogon im Chlathrat wurde durch Titration bestimmt und betrug 3,5%.

Beispiel 3

Man löst 41,75 g β-Cyclodextrin in 298 ml Wasser bei 80°C und tropft eine Lösung von 1,5 g (5E)-(16S)-13,14-Didehydro-1a,1b-dihomo-16,20-dimethyl-3-oxa-18,18,19,19-tetradehydro-6a-carba-prontagland in-I₂ in 24 ml Äthanol innerhalb von 15 Minuten zu. Man rührt 4 Stunden bei 60°C und läßt dann über Nacht unter Rühren abkühlen. Der ausgefallene Festsoff wird abgesaugt, mit 50 ml ainer Mischung aus Wasser/Äthanol (1 : 1) gewaschen und 24 Stunden bei 0,1 Torr und 25°C über Phosphorpentoxid getrocknet. Man erhält 38 g frei fließende Kristalle des β-Cyclodextrin-Clathrats des o.a. Carbacylinanalogons.

Der gehalt an Carbacyclinanalogon im Clathrat wurde durch Titration bestimmt und betrug 3,3%.

Beispiel 4

Man rührt 0,5 g (5E)-(16S)-13,14-Didehydro-16,20-dimethyl-18,18,19,19-tetradehydro-6a-carba prostaglandin-I₂ mit 15 g β-Cyclodextrin in 110 ml Wasser 50 Stunden bei 25°C. Der Feststoff wird abgesaugt, mit c. 10 ml einer Mischung aus Wasser/Äthanol (1 + 1) gewaschen und 24 Stunden bei 0,1 Torr und 25°C über Phosphorpentoxid getrocknet. Man erhält 11 g freifließende Kristalle des β-Cyclodextrin-Clathrats des o.a. Carbacyclinanalogons.

Der Gehalt an Carbacyclinanalogon im Clathrat wurde durch Titration bestimmt und betrug 3,6%.

Beispiel 5

Man löst 57,75 g β-Cyclodextrin bei 45°C in 1,53 1 Wasser, tropft innerhalb von 30 Minuten unter Rühren eine Lösung von 7,633 g (5E)-(16RS)-16-Methyl-18,18,19,19-tetradahydro-6a-carbaprostaglandin-$I_2$ in 45 ml Ethanol dazu und spült mit 5 ml Ethanol nach. Man kühlt innerhalb von 1 Stunde auf 25°C ab, rührt 2 Stunden bei dieser Temperatur und 3 Stunden im Eisbad. Man saugt das Kristallisat ab und wäscht mit eiskaltem Wasser, Aceton und wieder mit Wasser.

a) Man trocknet im Vakuum, bis das Kristallisat beginnt, bei Umgebungstemperatur und -luftfeuchtigkeit wieder Wasser aus der Luft aufzunehmen und läßt das Gewicht konstant werden. Man erhält 56,01 g (93,9%) eines freifließenden Kristallisats, das 6% Wasser enthält und einen durch Titration ermittelten Wirkstoffgehalt von 12,8% aufweist.

b) Man trocknet im Vakuum über Phosphorpentoxid und erhält 52,7 g eines freifließenden, hygroskopischen Kristallisats, das einen durch Titration ermittelten Wirkstoffgehalt von 13,6% aufweist.

### Beispiel 6

Man verseift 3,877 g (5E)-(16S)-13,14-Didehydro-16,20-dimethyl-3-oxa-18,18,19,19-tetradehydro-6a-carba-prostaglandin-$I_2$-tert.-butylester mit 11,7 ml 1n-Natronlauge, extrahiert Verunreinigungen mit Diethylether und verdünnt mit Wasser auf 660 ml Lösung. Man gibt 24,52 g D-Cyclodextrin zu und erwärmt unter Rühren auf 26°C bis zur Lösung. Man gibt innerhalb von 30 Minuten 11,7 ml 1n-Salzsäure dazu und rührt 90 Minuten. Man erwärmt auf 55°C, kühlt innerhalb von 1 Stunde auf 25°C ab und rührt 1 Stunde bei dieser Temperatur und 2,5 Stunden im Eisbad. Man saugt das Kristallisat ab, wäscht mit Eiswasser und trocknet wie im Beispiel 4 beschrieben.

a) Man erhält 22,53 g (89,2%) eines freifließenden Kristallisats, das 7,2% Wasser enthält und einen durch Titration ermittelten Wirkatoffgehalt von 13,36% aufweist.

b) Man erhält 21,0 g eines freifließenden, hygroskopischen Kristallisats, das einen durch Titration ermittelten Wirkstoffgehalt von 14,3% aufweist.

### Beispiel 7

Man löst 14,30 g β-Cyclodextrin bei 50°C in 205 ml Wasser, tropft innerhalb von 30 Minuten unter Rühren eine Lösung von 1,456 g (5E)-(16S)-13,14-Didehydro-1a,1b-dihomo-16,20-dimethyl-18,18,19,19-tetradehydro-6a-carba-prostaglandin-$I_2$ in 6 ml Ethanol dazu und spült mit 1,2 ml Ethanol nach. Man kühlt innerhalb von 1 Stunde auf 23°C ab, rührt 2 Stunden bei dieser Temperatur und 18 Stunden im Eisbad. Man saugt das Kristallinat ab, wäscht mit eiskaltem Wasser und trocknet wie im Beispiel 4 beschrieben.

a) Man erhält 14,155 g (95,8%) eines freifließenden Kristallisats, das 7,6% Wasser enthält und einen durch Titration ermittelten Wirkstoffgehalt von 9,9% aufweist.

b) Man erhält 13,1 g eines freifließenden, hygroskopischen Kristallisats, das einen durch Titration ermittelten Wirkstoffgehalt von 10,7% aufweist.

### Beispiel 8

(5E)-(16S)-13,14,18,18,19,19-Hexadehydro-16,20-dimethyl-6a-carba-prostaglandin-$I_2$-β-Cyclodextrinclathrat analog Beispiel 1 aus (5E)-(16S)-13,14,18,18,19,19-Hexadehydro-16,20-dimethyl-6a-carba-prostaglandin-$I_2$ und β-Cyclo-dextrinclathrat.

### Ansprüche

1. (5E)-(16S)-13,14-Didehydro-16,20-dimethyl-3-oxa-18,18,19,19-tetradehydro-6a-carba-prostaglandin-$I_2$-β-Cyclodextrin-Clathrat.

2. (5E)-(16S)-13,14-Didehydro-1a, 1b-dihomo-16,20-dimethyl-3-oxa-18,18, 19,19-tetradehydro-6a-carba-prostaglandin-$I_2$-β-Cyclodextrin-Clathrat.

3. (5E)-(16 S)-13,14,18,18,19,19-Hexadehydro-16,20-dimethyl-6a-carba-prostaglandin-$I_2$-β-Cyclodextrinclathrat.

4. (5E)-(16RS)-16-Methyl-18,18,19,19-tetradehydro-6a-carba-prostaglandin-$I_2$-β-Cyclodextrin-Clathrat.

5. (5E)-(16S)-13,14-Didehydro-1a,1b-dihomo-16,20-dimethyl-18,18,19,19-tetradehydro-6a-carba-prostaglandin $I_2$-β-Cyclodextrin-Clathrat.

### Claims

1. (5E)-(16S)-13,14-didehydro-16,20-dimethyl-3-oxa-18,18,19,19-tetradehydro-6a-carbaprostagland in $I_2$ β-cyclodextrin clathrate.

2. (5E)-(16S)-13,14-didehydro-1a,1b-dihomo-16,20-dimethyl-3-oxa-18,18,19,19-tetradehydro-6a-carbaprostaglandin $I_2$ β-cyclodextrin clathrate.

3. (5E)-(16S)-13,14,18,18,19,19-hexadehydro-16,20-dimethyl-6a-carbaprostaglandin $I_2$-β-cyclodextrin clathrate.

4. (5E)-(16RS)-16-methyl-18,18,19,19-tetradehydro-6a-carbaprostaglandin $I_2$-β -cyclodextrin clathrate.

5. (5E)-(16S)-13,14-didehydro-1a,1b-dihomo-16,20-dimethyl-18,18,19,19-tetra-dehydro-6a-carba-prostaglandin $I_2$-β-cyclodextrin-clathrate.

### Revendications

1. β-cyclodextrine-clathrate de la didéhydro-13,14 diméthyl-16,20 oxa-3 tétradéhydro-18,18,19,19 carba-6a prostaglandine I$_2$ (5E)-(16S).

2. β-cyclodextrine-clathrate de la didéhydro-13,14 dihomo-1a,1b diméthyl-16,20 oxa-3 tétradéhydro-18,18,19,19 carba-6a prostaglandine I$_2$ (5E) (16S).

3. β-cyclodextrine-clathrate de l'hexadéhydro-13,14,18,18 19,19 diméthyl-16,20 carba-6a prostaglandine I$_2$-(5E)-(16S).

4. β-Cyclodextrine-clathrate de la méthyl-16 tétradéhydro-18,18,19,19 carba-6a prostaglandine I$_2$ (5E)-(16RS).

5. β-cyclodextrine-clathrate de la didéhydro-13,14 dihomo-1a,1b diméthyl-16,20 tétradéhydro-18,18,19,19 carba-6a prostaglandine I$_2$ (5E)-(16S).